# EUROPEAN PATENT APPLICATION

(11) **EP 4 534 549 A1**
(43) Date of publication of application: **09.04.2025**
(21) Application number: 22947806.0
(22) Date of filing: 29.12.2022
(51) Int. Cl.: C07K 14/245, C12N 15/31, C12N 1/21, C12P 13/08

(54) **MREC MUTANT AND USE THEREOF IN L-VALINE FERMENTATIVE PRODUCTION**

(30) Priority: 22.06.2022 CN 202210710799
(71) Applicant: Anhui Huaheng Biotechnology Co., Ltd., Hefei, Anhui 231131 (CN); Tianjin Institute of Industrial Biotechnology, Chinese Academy of Sciences, Tianjin 300308 (CN)
(72) Inventor: ZHANG, Xueli, Tianjin 300308 (CN); GUO, Henghua, Hefei, Anhui 231131 (CN); LIU, Pingping, Tianjin 300308 (CN); ZHANG, Dongzhu, Hefei, Anhui 231131 (CN); TANG, Jinlei, Tianjin 300308 (CN); LIU, Shupeng, Hefei, Anhui 231131 (CN)
(74) Representative: Delbarba, Andrea
(86) International application number: PCT/CN2022/143630
(87) International publication number: WO 2023/246071

(57) **Abstract**

The invention provides an mreC mutant and use thereof in L-valine fermentation production. The mreC mutant is a protein obtained by mutating a proline at the 150-th site of a wild-type mreC amino acid sequence into a leucine; and by transferring a point mutation of mreC into a microbial genome, namely C at the 449-th site of a nucleotide sequence in an encoding region of a wild-type mreC gene is mutated into T, the production capacity and biomass of a valine engineering strain may be significantly improved, and by the knockout and introduction of relevant enzyme genes in an L-valine anaerobic fermentation pathway, appropriate carbon metabolic flow and reducing power balance control in the production process of L-valine are obtained. By the regulation and control of multi-gene, a suitable metabolic pathway for L-valine fermentation is formed by coordination, and the high-level synthesis of the L-valine in microorganisms is achieved.

## Description

### Technical Field

The present disclosure belongs to the field of bioengineering technologies, and specifically relates to an mreC mutant and use thereof in production of L-valine, in particular to use of an mreC mutant and a nucleic acid molecule thereof in construction of L-valine engineering bacteria and production of L-valine.

### Background

L-valine is one of 20 amino acids that make up proteins, and is also one of essential amino acids and glycogenic amino acids in mammals. The L-valine has important applications in the fields of feeds, foods, medicines, cosmetics, antibiotics, and herbicides and the like, especially in the field of feeds. In 2018, the minimum limit level of the L-valine was specifically added in the group standard T/CFIAS 001-2018 "Formula Feed for Piglets, Growing and Finishing Pigs" drafted by the China Feed Industry Association; in 2017-2019, the L-valine showed a compound annual growth rate of nearly 100%; and therefore, in the future, the application and demand of the L-valine in the feed field may be larger and larger inevitably, and the market potential is unlimited. The L-valine may also be used as a food additive, a nutritional supplement, and a flavoring agent and the like, so it also has a wide range of applications in the field of foods and medicines. With the rapid development of the L-valine from traditional feeds additives to high value-added industries such as the foods, medicines, and cosmetics, and under the guidance of national policies, the market demand for the L-valine is bound to lead to the breakthrough growth, and the L-valine may become the next limiting amino acid in pig and poultry daily ration. According to data from the China Biotech Fermentation Industry Association, the global demand for valine was about 32500 tons in 2019, with a future growth rate of 24%, indicating a huge market potential.

With the rapid development of synthetic biology and metabolic engineering, the technology to produce bulk chemicals, fine chemicals, and natural products from renewable resources through microbial fermentation is increasingly valued for its green, environmentally friendly, and efficient production, and have gradually become an important force to substitute petroleum based chemicals, showing a vigorous development trend. At present, the L-valine is mainly produced by a fermentation method. At present, the fermentation production of L-valine is mostly achieved by aerobic or two-step fermentation, with high energy consumption and low conversion rate. Compared to the aerobic fermentation, an anaerobic process does not require aeration during the production process, and the conversion rate of products is usually close to the theoretical maximum value. This results in the advantages of low energy consumption and high conversion rate, making it a research hotspot in recent years. The anaerobic fermentation synthesis of L-valine by wild-type strains is significantly limited due to extensive intracellular feedback inhibition and other regulatory networks, leading to extremely low yields of L-valine.

In allusion to the above technical drawbacks, existing technologies have involved strain modification for the anaerobic fermentation process of the valine, including the introduction and knockout of genes for enzymes related to the metabolic pathway to address cofactor imbalances, thereby the efficient production of the L-valine by recombinant strains is achieved under anaerobic conditions. However, the fermentation capacity of the above recombinant strains for producing L-valine still needs to be improved without domestication.

### Summary

The purpose of the present disclosure is to provide an mreC mutant and use thereof in fermentation production of L-valine. The present disclosure may significantly improve the production capacity and biomass of valine engineering strains by transferring a genomic mutation into a microbial genome that may significantly improve the growth and valine yield of the engineering strains.

In a first aspect, the present disclosure provides an mreC mutant, which is a protein composed of the amino acid sequence shown in SEQ ID NO: 3.

Further, the mreC mutant is a protein obtained after the proline at the 150-th site of the EcoIC_0457 rod-shaped determining protein amino acid sequence shown in SEQ ID NO: 2 is mutated into leucine.

In a second aspect, the present disclosure provides a nucleic acid molecule encoding the above mreC mutant.

Further, the nucleic acid molecule is any one of deoxyribonucleic acid (DNA) molecules in (a1)-(a2):
(a1) a DNA molecule of which an encoding region is shown in SEQ ID NO: 4; and
(a2) a DNA molecule of which a nucleotide sequence is shown in SEQ ID NO: 4.

Herein, the DNA molecule shown in SEQ ID NO: 4 is that a point mutation is transferred at the 449-th site of the nucleotide sequence of the encoding region of the wild-type mreC gene shown in SEQ ID NO: 1, mutating C to T.

In a third aspect, the present disclosure provides a biomaterial, which is any one of the following (b1)-(b3):
(b1) a gene expression cassette containing the above nucleic acid molecule;
(b2) a recombinant vector containing the above nucleic acid molecule; and
(b3) a recombinant microorganism containing the above nucleic acid molecule.

Further, the recombinant vector may be a recombinant plasmid obtained by inserting the nucleic acid molecule into an expression vector or a cloning vector.

Further, the recombinant microorganism may be a microorganism containing the above recombinant vector; and the microorganism may be yeast, bacteria, algae, or fungi. Further, the bacteria may be *Escherichia coli.*

The present disclosure provides use of any one of the following (c1)-(c2):
(c1) use of the above mreC mutant or nucleic acid molecule or biomaterial in construction of L-valine engineering bacteria; and
(c2) use of the above mreC mutant or nucleic acid molecule or biomaterial in L-valine production.

The present disclosure provides a method for constructing an L-valine engineering bacteria, including the following steps: transferring the above nucleic acid molecule into a microorganism.

Herein, the microorganism is any microorganism in the existing technologies that has the ability to produce the L-valine and involves the rod-shaped determining protein mreC in the L-valine production pathway; due to its clear genetic background, convenient genetic manipulation tools, extensive substrate utilization, easy culture, rapid growth and other advantages, *Escherichia coli* has become one of the most important model strains in metabolic engineering transformation already, and has achieved industrialization in fermentation production of compounds such as L-alanine, D-lactic acid, and succinic acid. Therefore, it is preferred to choose the *Escherichia coli,* the *Escherichia coli* may be a wild type, and may also be any mutant type that does not affect the production of L-valine; *Escherichia coli* MG1655 is considered to be a biosafety microbial strain, therefore it is more preferred.

During anaerobic fermentation, glucose metabolism is mainly achieved by the glycolytic pathway, where the metabolism of 1 mol of glucose produces 2 mol of pyruvate, along with the generation of 2 mol of adenosine triphosphate (ATP) and 2 mol of nicotinamide adenine dinucleotide (NADH), leading to a redox imbalance in the modified *Escherichia coli* under anaerobic conditions, namely NADH is excessive, but nicotinamide adenine dinucleotide phosphate (NADPH) supply is insufficient.

In order to solve the problem of redox imbalance during the anaerobic fermentation and achieve the efficient production of L-valine under the anaerobic conditions, the present disclosure further modifies the above recombinant microorganism by any one of the following modifications (d1)-(d2):
(d1) transferring a NADH-dependent acetohydroxy acid isoreductase gene *kari* and a NADH-dependent leucine dehydrogenase gene *leuDH* into the above recombinant microorganism, thereby enhancing the enzyme activity; and
(d2) transferring a NADPH-dependent acetohydroxy acid isomeroreductase gene *ilvC* and a NADH-dependent leucine dehydrogenase gene *leuDH* into the above recombinant microorganism, and activating the activity of a transhydrogenase PntAB and a NAD kinase YfjB, thereby enhancing the enzyme activity.

The "transfer" may be present in the microorganism in any suitable manners known in this field, such as in the form of a plasmid or in the form of integration into a genome.

In one embodiment, the enzyme encoding gene integrated into the genome is placed under the control of suitable regulatory elements.

In one embodiment, a plasmid containing an enzyme encoding gene and a regulatory element is transferred into the microorganism.

The "activation" may be achieved by any manners known in this field, such as placing the enzyme encoding gene to be activated under the control of the suitable regulatory elements to enhance the expression of the above enzyme encoding gene.

The regulatory element may be inserted into the upstream of a target gene by a known genetic engineering method. The method includes, but is not limited to, a mode of gene recombination, such as a mode of homologous recombination, in which the regulatory element is inserted into the upstream of the target gene encoding sequence, so that the intensity of target gene expression is enhanced.

The regulatory element may be selected from an M1-93 artificial regulatory element, an M1-37 artificial regulatory element, an M1-46 artificial regulatory element, or a RBS5 artificial regulatory element.

In one embodiment, the M1-93 artificial regulatory element regulates an acetolactate synthase gene *ilvBN,* an acetolactate synthase gene *ilvGM*, a leucine dehydrogenase encoding gene *leuDH,* a dihydroxy acid dehydratase encoding gene *ilvD,* and a transhydrogenase encoding gene *pntAB.*

In one embodiment, the M1-37 artificial regulatory element regulates a NAD kinase encoding gene *yfjB.*

In one embodiment, the M1-46 artificial regulatory element regulates a NADPH-dependent acetohydroxy acid isomeroreductase encoding gene *ilvC.*

In one embodiment, the RBS5 artificial regulatory element regulates a NADH-dependent acetohydroxy acid isoreductase encoding gene *kari.*

Furthermore, it further includes the following modifications (1)-(6) to the recombinant microorganism:
(1) knocking out a lactate dehydrogenase gene *IdhA;*
(2) knocking out a phosphoacetyl transferase gene pta and an acetate kinase gene *ackA;*
(3) knocking out an alcohol dehydrogenase gene *adhE*;
(4) knocking out a fumarate reductase gene frd and a pyruvate formate lyase gene *pflB*;
(5) knocking out a methylglyoxal synthase gene *mgsA*; and
(6) enhancing the activities of an acetolactate synthase (AHAS) and a dihydroxy acid dehydratase (IIvD).

Those skilled in the field may understand that gene knockout may be performed using known techniques to reduce or inactivate the activity of the enzyme. The knockout operation is aimed at an endogenous enzyme gene of the original microorganism, resulting in the reduction or inactivation of the above endogenous enzyme in the microorganism.

In one embodiment, the transfer, mutation, or knockout of the target gene is achieved by integrating them into the genome of the microorganism.

In one embodiment, the transfer, mutation, or knockout of the enzyme gene is achieved by using a homologous recombination method.

In one embodiment, the transfer, mutation, or knockout of the enzyme gene is achieved by using a two-step homologous recombination method.

Transferring, mutating, or knocking-out the target gene by using the two-step homologous recombination method includes the following steps:
(1) Preparation of DNA fragment I: Using pXZ-CS plasmid (Tan, et. al., Appl Environ Microbiol, 2013, 79:4838-4844) DNA as a template, DNA Fragment I is amplified with primer 1 for the first step of homologous recombination.
(2) First step of homologous recombination: a pKD46 plasmid (Datsenko and Wanner 2000, Proc Natl Acad Sci USA 97:6640-6645) is transformed into an original strain, and then the DNA fragment I is transformed to the original strain with pKD46; and a single colony correctly verified by a colony polymerase chain reaction (PCR) is named as recombinant bacteria 1.
(3) Preparation of DNA fragment II: Using the genomic DNA as a template, DNA fragment II is amplified with primer 2 for the second step of homologous recombination.
(4) Second step of homologous recombination: the DNA fragment II is transformed into the recombinant bacteria 1, and a single colony correctly verified by the colony PCR is named as recombinant bacteria 2.

The "substitution" may be performed in a mode known to those skilled in the field, for example, the encoding sequence of a gene to be inserted is integrated into a substituted gene encoding sequence site in the microbial chromosome, so that the gene encoding sequence in the original site is substituted by the encoding sequence of the integrated inserted gene.

Further, genetic engineering modes such as the homologous recombination may also be used to substitute the encoding sequence of the enzyme gene in the above (1)-(5) with the encoding sequence of another gene, thereby the activity of the above endogenous enzyme of the microorganism is reduced or inactivated. The gene that substitutes these endogenous enzymes may be a gene to be expression-enhanced, such as the above *ilvC* gene or *leuDH* gene.

In one embodiment, the knockout of the item (4) is achieved by substituting the endogenous frd gene of the microorganism with the *leuDH* gene; and in one embodiment, the knockout of the item (4) is achieved by substituting the endogenous *pflB* gene of the microorganism with the *ilvD* gene.

In one embodiment, the knockout of the item (5) is achieved by substituting the endogenous *mgsA* gene of the microorganism with the *ilvC* gene.

Herein, the acetolactate synthase AHAS includes ilvBN, ilvGM, and ilvlH.

Preferably, the activity of ilvlH is enhanced by relieving the feedback inhibition of valine on ilvH; and more preferably, a mutation is transferred in the *ilvH* gene to relieve the feedback inhibition of L-valine.

For the involved acetolactate synthase biologically synthesized by the L-valine, in addition to an isozyme II (herein also referred to as AHAS II), an isozyme III (herein also referred to as AHAS III) is also known. The AHASIII is encoded by an ilvlH operon, and the operon is formed by ilvl encoding a large subunit (catalytic subunit) and *ilvH* encoding a small subunit (control subunit). The AHASIII is subjected to the feedback inhibition of the L-valine. reported methods may be used to mutate the ilvl gene, such as amino acid substitution of ilvH 14 Gly→Asp (Vyazmensky, M. and the like, "Biochemistry" 35:10339-10346 (1996) ) and/or ilvH 17Ser→Phe (US6737255B2); and ilvH612 (De Felice and the like, "Journal of Bacteriology" 120:1058-1067 (1974) ) and the like.

In one embodiment, the activity of the dihydroxy acid dehydratase *ilvD* is enhanced by transferring the *ilvD* gene into the microorganism.

The present disclosure provides L-valine engineering bacteria obtained by using the above construction method.

In one embodiment, the L-valine engineering bacteria are recombinant *Escherichia coli* obtained by transferring a mreC mutation point into the genome (transferring the point mutation at the 449-th site in the encoding region of the wild-type mreC gene, C is mutated into T, and the amino acid at the 150-th site is correspondingly changed from proline (P) to leucine (L) ).

In one embodiment, the L-valine engineering bacteria are recombinant *Escherichia coli* obtained by transferring the above mreC mutation point into the genome, and transferring the NADH-dependent acetohydroxy acid isoreductase gene *kari* and the NADH-dependent leucine dehydrogenase gene *leuDH* into the engineering bacteria.

In one embodiment, the L-valine engineering bacteria are recombinant *Escherichia coli* obtained by transferring the above mreC mutation point into the genome, transferring the NADPH-dependent acetohydroxy acid isomeroreductase gene *ilvC* and the NADH-dependent leucine dehydrogenase gene *leuDH* into the engineering bacteria, and activating the activities of the transhydrogenase PntAB and the NAD kinase YfjB.

The present disclosure provides use of the above L-valine engineering bacteria in L-valine production.

Herein, the L-valine production is aerobic fermentation culture, microaerobic fermentation culture, or anaerobic fermentation culture, preferably the anaerobic fermentation culture.

The present disclosure provides a method for producing an L-valine, including the following steps:
(1) culturing the above L-valine engineering bacteria; and
(2) separating and harvesting the L-valine.

Herein, the fermentation culture is aerobic, microaerobic, or anaerobic fermentation culture, preferably the anaerobic fermentation culture.

In one embodiment, the anaerobic fermentation culture includes the following steps:
(1) seed culture: a clone on a plate is picked and inoculated into a seed culture medium, and cultivated at 37°C with shaking to obtain seed culture solution; and
(2) fermentation culture: the seed culture solution is inoculated into the fermentation medium and cultivated at 37°C with shaking to obtain fermentation solution. PH of a fermentation tank is controlled to 7.0. Any gases are not allowed in a culture process.

Herein the seed culture medium is formed by the following components (a solvent is water):
Macroelement: 20 g/L of glucose, 0.87 g/L of NH₄H₂PO₄, 2.63 g/L of (NH₄) ₂HPO₄, 0.18 g/L of MgSO₄·7H₂O, and 0.15 g/L of betaine-HCl.
Microelement: 1.5 µg/L of FeCl₃.6H₂O, 0.1 µg/L of CoCl₂·6H₂O, 0.1 µg/L of CuCl₂·2H₂O, 0.1 µg/L of ZnCl₂, 0.1 µg/L of Na₂MoO₄·2H₂O, 0.2 µg/L of MnCl₂·4H₂O, and 0.05 µg/L of H₃BO₃.

The fermentation culture medium and the seed culture medium have the same components, and a difference is only that the glucose concentration is 50 g/L.

Compared with the existing technologies, the present disclosure has the following beneficial technical effects: by transferring the point mutation of mreC into the microbial genome, the present disclosure effectively improves the fermentation ability of the L-valine engineering bacteria, increases the yield of the L-valine by its fermentation, and significantly increases the cell biomass. In addition, by the knockout and introduction of relevant enzyme genes in an L-valine anaerobic fermentation pathway, the problem of imbalance of reducing power in anaerobic metabolism is effectively solved, and appropriate carbon metabolic flow and reducing power balance control in the production process of L-valine are obtained. By the regulation and control of multi-gene, a suitable metabolic pathway for L-valine fermentation is formed by coordination, and the high-level production of the L-valine in microorganisms is achieved by the one-step anaerobic fermentation method. It also reduces the production cost, and has the significant application value.

### Brief Description of the Drawings

Drawings of the description for constituting a part of the present application are used to provide further understanding of the present disclosure. Exemplary embodiments of the present disclosure and descriptions thereof are used to explain the present disclosure, and do not constitute improper limitation to the present disclosure. In the drawings:

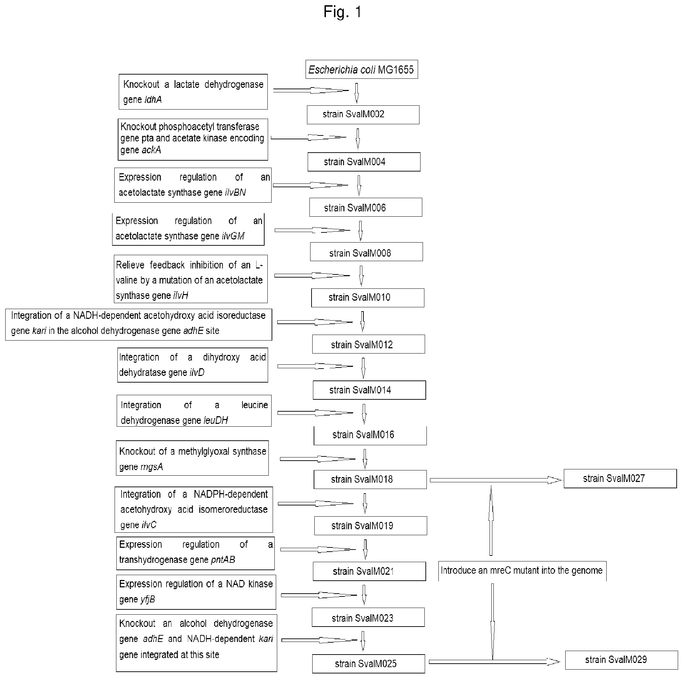

Fig. 1 shows the construction process of L-valine engineering bacteria.

### Detailed Description of the Embodiments

The present disclosure is further described by the following embodiments, but any embodiments or combinations thereof should not be interpreted as limiting a scope or an embodiment of the present disclosure. The scope of the present disclosure is defined by appended claims. In combination with the description and common knowledge in the field, those of ordinary skill in the field may clearly understand the scope defined by the claims. Without departing from the spirit and scope of the present disclosure, those skilled in the field may make any modifications or changes to technical schemes of the present disclosure, and such modifications and changes are also included in the scope of the present disclosure.

Experimental methods used in the following embodiments are conventional methods unless otherwise specified. Materials, reagents and the like used in the following embodiments may be obtained from commercial sources unless otherwise specified.

Due to its clear genetic background, convenient genetic manipulation tools, extensive substrate utilization, easy culture, rapid growth and other advantages, *Escherichia coli* becomes one of the most important model strains in metabolic engineering transformation already, and achieves industrialization in production of compounds such as L-alanine, D-lactic acid, and succinic acid by using engineering strains. *Escherichia coli* MG1655 is considered to be a biosafety microbial strain, and compounds produced by cell factories constructed using this strain may have great application potential in the fields of medicines, foods and the like. The inventors of the present disclosure screens out a batch of recombinant strains capable of producing an L-valine efficiently by the early domestication of strains, and by genome sequencing of the original strain and this batch of the recombinant strains, it is found that a point mutation occurs in a mreC gene, encoding an EcoIC_0457 rod-shaped determining protein, in the genomes of some recombinant strains. Therefore, the introduction of the point mutation in the mreC gene into the microbial genome may be of great significance for the construction of L-valine producing strains and the efficient fermentation of L-valine. In the following embodiments, the *Escherichia coli* MG1655 is used as the original strain, a genomic mutation (point mutation of mreC) that may significantly improve the growth and L-valine production of the engineering strains (producing strain obtained by modifications of *Escherichia coli* MG1655) is provided. The mreC gene encodes the EcoIC_0457 rod-shaped determining protein, and by transferring this point mutation into the genome of the producing strain obtained by the modifications of *Escherichia coli* MG1655, the production capacity and biomass of the L-valine engineering strain may be significantly improved.

Strains and plasmids used in this research are specifically shown in Table 1, and primers used are specifically shown in Table 2.

**Table 1: Strains and plasmids used in the present disclosure**

| Strain | Related characteristics | Source |
|---|---|---|
| MG1655 | Wild type | Laboratory preservation |
| M1-93 | ATCC8739, FRT-Km-FRT::M1-93::lacZ | Lu, et. al., Appl Microbiol Biotechnol, 2012, 93:2455-2462 |
| M1-46 | ATCC8739, FRT-Km-FRT::M1-46::lacZ | Lu, et. al., Appl Microbiol Biotechnol, 2012, 93:2455-2462 |
| SvalM001 | MG1655, IdhA::cat-sacB | Constructed by the present disclosure |
| SvalM002 | SvalM001, ΔIdhA | Constructed by the present disclosure |
| SvalM003 | SvalM002, ackA-pta::cat-sacB | Constructed by the present disclosure |
| SvalM004 | SvalM003, Δ ackA-pta | Constructed by the present disclosure |
| SvalM005 | SvalM004, cat-sacB::ilvB | Constructed by the present disclosure |
| SvalM006 | SvalM005, M1-93:: ilvB | Constructed by the present disclosure |
| SvalM007 | SvaIM006, cat-sacB::ilvG | Constructed by the present disclosure |
| SvalM008 | SvalM007, M1-93:: ilvG | Constructed by the present disclosure |
| SvalM009 | SvalM008, ilvH::cat-sacB | Constructed by the present disclosure |
| SvalM010 | SvaIM009, ilvH:: ilvH* | Constructed by the present disclosure |
| SvalM011 | SvalM010, adhE::cat-sacB | Constructed by the present disclosure |
| SvalM012 | SvalM011, adhE::RBS5-kari | Constructed by the present disclosure |
| SvalM013 | SvalM012, pfIB::cat-sacB | Constructed by the present disclosure |
| SvalM014 | SvalM013, pfIB::RBS4-ilvD | Constructed by the present disclosure |
| SvalM015 | SvalM014, frd::cat-sacB | Constructed by the present disclosure |
| SvalM016 | SvalM015, frd::M1-93-leuDH | Constructed by the present disclosure |
| SvalM017 | SvalM016, mgsA::cat-sacB | Constructed by the present disclosure |
| SvalM018 | SvalM017, ΔmgsA | Constructed by the present disclosure |
| SvalM019 | SvalM018, mgsA::M1-46-ilvC | Constructed by the present disclosure |
| SvalM020 | SvalM019, cat-sacB::pntAB | Constructed by the present disclosure |
| SvalM021 | SvalM020, M1-93-pntAB | Constructed by the present disclosure |
| SvalM022 | SvalM021, cat-sacB::yfjB | Constructed by the present disclosure |
| SvalM023 | SvalM022, M1-37-yfjB | Constructed by the present disclosure |
| SvalM024 | SvalM023, adhE::cat-sacB | Constructed by the present disclosure |
| SvalM025 | SvalM024, ΔadhE | Constructed by the present disclosure |
| SvalM026 | SvalM018, mreC::cat-sacB | Constructed by the present disclosure |
| SvalM027 | SvalM026, mreC::mreC* | Constructed by the present disclosure |
| SvalM028 | SvalM025, mreC::cat-sacB | Constructed by the present disclosure |
| SvalM029 | SvalM028, mreC::mreC* | Constructed by the present disclosure |
| Plasmid | Related character | Source |
| pUC57-M1-93-leuDH | Artificial regulatory element M1-93 and chemically synthesized gene *leuDH* are linked to a pUC57 vector together | Nanjing Genscript Biotechnology Co., Ltd. |
| pUC57-RBS5-kari | Artificial regulatory element RBS5 and chemically synthesized gene *kari* are linked to a pUC57 vector together | Nanjing Genscript Biotechnology Co., Ltd. |

**Table 2: Primers and sequence information used in the present disclosure**

| Primer name | Sequence |
|---|---|
| IdhA-cs-up (SEQ ID NO:5) | ttcaacatcactggagaaagtcttatgaaactcgccgtttatagcacaaaTGTGACGG AAGATCACTTCGCAG |
| IdhA-cs-down (SEQ ID NO:6) | ttcaacatcactggagaaagtcttatgaaactcgccgtttatagcacaaaTGTGACGG AAGATCACTTCGCAG agcggcaagattaaaccagttcgttcgggcaggtttcgcctttttccagaTTATTTGTT AACTGTTAATTGTCCT |
| XZ-IdhA-up (SEQ ID NO:7) | GATAACGGAGATCGGGAATG |
| XZ-IdhA-down (SEQ ID NO:8) | CTTTGGCTGTCAGTTCACCA |
| IdhA-del-down (SEQ ID NO:9) | agcggcaagattaaaccagttcgttcgggcaggtttcgcctttttccagatttgtgctataaac ggcgagt |
| ackA-cs-up (SEQ ID NO:10) | agcggcaagattaaaccagttcgttcgggcaggtttcgcctttttccagatttgtgctataaac ggcgagt aggtacttccatgtcgagtaagttagtactggttctgaactgcggtagttTGTGACGGA AGATCACTTCGCAG ggtcggcagaacgctgtaccgctttgtaggtggtgttaccggtgttcagaTTATTTGTT AACTGTTAATTGTCCT |
| pta-cs-down (SEQ ID NO:11) | ggtcggcagaacgctgtaccgctttgtaggtggtgttaccggtgttcagaTTATTTGTT AACTGTTAATTGTCCT |
| XZ-ackA-up (SEQ ID NO:12) | cgggacaacgttcaaaacat |
| XZ-pta-down (SEQ ID NO:13) | attgcccatcttcttgttgg |
| ackA-del-down (SEQ ID NO:14) | ggtcggcagaacgctgtaccgctttgtaggtggtgttaccggtgttcagaaactaccgcagt tcagaacca |
| ilvB pro-catup (SEQ ID NO:15) | ggtcggcagaacgctgtaccgctttgtaggtggtgttaccggtgttcagaaactaccgcagt tcagaacca ctgacgaaacctcgctccggcggggttttttgttatctgcaattcagtacTGTGACGGA AGATCACTTCGCA tctgcgccggtaaagcgcttacgcgtcgatgttgtgcccgaacttgccatTTATTTGTT |
| ilvB pro-catdown | tctgcgccggtaaagcgcttacgcgtcgatgttgtgcccgaacttgccatTTATTTGTT |
| (SEQ ID NO:16) | AACTGTTAATTGTCCT |
| ilvB pro-up (SEQ ID NO:17) | AACTGTTAATTGTCCT ctgacgaaacctcgctccggcggggttttttgttatctgcaattcagtacTTATCTCTGG CGGTGTTGAC |
| ilvB pro-down (SEQ ID NO:18) | tctgcgccggtaaagcgcttacgcgtcgatgttgtgcccgaacttgccatAGCTGTTT CCTGGTTTAAAC |
| ilvB pro-YZup (SEQ ID NO:19) | gttctgcgcggaacacgtatac |
| ilvB pro-YZdown (SEQ ID NO:20) | ccgctacaggccatacagac |
| ilvG pro-catup (SEQ ID NO:21) | tgaactaagaggaagggaacaacattcagaccgaaattgaatttttttcaTGTGACG GAAGATCACTTCGCA |
| ilvG pro-catdown (SEQ ID NO:22) | ttcacaccctgtgcccgcaacgcatgtaccacccactgtgcgccattcatTTATTTGTT AACTGTTAATTGTCCT |
| ilvG pro-up (SEQ ID NO:23) | tgaactaagaggaagggaacaacattcagaccgaaattgaatttttttcaTTATCTCT GGCGGTGTTGAC |
| ilvG pro-down (SEQ ID NO:24) | ttcacaccctgtgcccgcaacgcatgtaccacccactgtgcgccattcatAGCTGTTT CCTGGTTTAAACG |
| ilvG pro-YZup (SEQ ID NO:25) | gcataagatatcgctgctgtag |
| ilvG p-YZdown (SEQ ID NO:26) | gccagttttgccagtagcac |
| ilvH*-cat-up (SEQ ID NO:27) | agaacctgattatgCGCCGGATATTATCAGTCTTACTCGAAAATGAAT CATGTGACGGAAGATCACTTCGCA TTCATCGCCCACGGTCTGGATGGTCATACGCGATAATGTCGG |
| ilvH*-cat-down | TTCATCGCCCACGGTCTGGATGGTCATACGCGATAATGTCGG |
| (SEQ ID NO:28) | ATCGTCGGTTATTTGTTAACTGTTAATTGTCCT |
| ilvH*-mut-up (SEQ ID NO:29) | ATCGTCGGTTATTTGTTAACTGTTAATTGTCCT agaacctgattatgCGCCGGATATTATCAGTCTTACTCGAAAATGAAT CAGaCGCGTTATtCCGCGTGATTGGC |
| ilvH*-mut-down (SEQ ID NO:30) | CACACCAGAGCGAGCAACCTC |
| ilvH*-mutYZ-up (SEQ ID NO:31) | atgagctggaaagcaaacttagc |
| adhE-cs-up (SEQ ID NO:32) | ATAACTCTAATGTTTAAACTCTTTTAGTAAATCACAGTGAGTGT GAGCGCTGTGACGGAAGATCACTTCGCA |
| adhE-cs-down (SEQ ID NO:33) | CCGTTTATGTTGCCAGACAGCGCTACTGATTAAGCGGATTTTT TCGCTTTTTATTTGTTAACTGTTAATTGTCCT |
| XZ-adhE-up (SEQ ID NO:34) | CATGCTAATGTAGCCACCAAA |
| XZ-adhE-down (SEQ ID NO:35) | TTGCACCACCATCCAGATAA |
| adhE-RBS5-up (SEQ ID NO:36) | ATAACTCTAATGTTTAAACTCTTTTAGTAAATCACAGTGAGTGT GAGCGCTTATCTCTGGCGGTGTTGAC |
| adhE-kari-down (SEQ ID NO:37) | CCGTTTATGTTGCCAGACAGCGCTACTGATTAAGCGGATTTTT TCGCTTTTTAGATAACTTTTTTCTTCA |
| pfIB-CS-up (SEQ ID NO:38) | aaacgaccaccattaatggttgtcgaagtacgcagtaaataaaaaatccaTGTGAC GGAAGATCACTTCGCAG |
| pfIB-CS-down (SEQ ID NO:39) | CGGTCCGAACGGCGCGCCAGCACGACGACCGTCTGGGGTG TTACCCGTTTTTATTTGTTAACTGTTAATTGTCCT aaacgaccaccattaatggttgtcgaagtacgcagtaaataaaaaatccaTTATCTC |
| RBSL4-pfIB-up | aaacgaccaccattaatggttgtcgaagtacgcagtaaataaaaaatccaTTATCTC |
| (SEQ ID NO:40) | TGGCGGTGTTGAC |
| RBSL4-ilvD-down (SEQ ID NO:41) | gcggaacggtacttaggcatTGCTGACCTCCTGGTTTAAAC |
| ilvD-RBSL4-up (SEQ ID NO:42) | GTTTAAACCAGGAGGTCAGCAatgcctaagtaccgttccgc |
| ilvD-pflB-down (SEQ ID NO:43) | CGGTCCGAACGGCGCGCCAGCACGACGACCGTCTGGGGTG TTACCCGTTTttaaccccccagtttcgatttatc |
| XZ-pfIB-up600 (SEQ ID NO:44) | CTGCGGAGCCGATCTCTTTAC |
| XZ-pflB-down (SEQ ID NO:45) | CGAGTAATAACGTCCTGCTGCT |
| XZ-frd-up (SEQ ID NO:46) | TGCAGAAAACCATCGACAAG |
| Xz-frd-down (SEQ ID NO:47) | CACCAATCAGCGTGACAACT |
| frd-cs-up (SEQ ID NO:48) | GAAGGCGAATGGCTGAGATGAAAAACCTGAAAATTGAGGTGG TGCGCTATTGTGACGGAAGATCACTTCGCA |
| frd-cs-down (SEQ ID NO:49) | TCTCAGGCTCCTTACCAGTACAGGGCAACAAACAGGATTACG ATGGTGGCTTATTTGTTAACTGTTAATTGTCCT |
| frd-M93-up (SEQ ID NO:50) | GAAGGCGAATGGCTGAGATGAAAAACCTGAAAATTGAGGTGG TGCGCTATTTATCTCTGGCGGTGTTGAC |
| frd-leuDH-down (SEQ ID NO:51) | TCTCAGGCTCCTTACCAGTACAGGGCAACAAACAGGATTACG ATGGTGGCTTAACGGCCGTTCAAAATATTTTTTTC gtaggaaagttaactacggatgtacattatggaactgacgactcgcacttTGTGACG |
| mgsA-cs-up | gtaggaaagttaactacggatgtacattatggaactgacgactcgcacttTGTGACG |
| (SEQ ID NO:52) | GAAGATCACTTCGCAG |
| mgsA-cs-down (SEQ ID NO:53) | GAAGATCACTTCGCAG gcgtttgccacctgtgcaatattacttcagacggtccgcgagataacgctTTATTTGTT AACTGTTAATTGTCCT |
| XZ-mgsA-up (SEQ ID NO:54) | cagctcatcaaccaggtcaa |
| XZ-mgsA-down (SEQ ID NO:55) | aaaagccgtcacgttattgg |
| mgsA-del-down (SEQ ID NO:56) | gcgtttgccacctgtgcaatattacttcagacggtccgcgagataacgctaagtgcgagtc gtcagttcc |
| M46-mgsA-up (SEQ ID NO:57) | gtaggaaagttaactacggatgtacattatggaactgacgactcgcacttTTATCTCT GGCGGTGTTGAC |
| M46-ilvC-down (SEQ ID NO:58) | gtGTATTGAAGTAGTTAGCCATAGCTGTTTCCTGGTTTAAACC |
| ilvC-M46-up (SEQ ID NO:59) | GGTTTAAACCAGGAAACAGCTATGGCTAACTACTTCAATACac |
| ilvC-mgsA-down (SEQ ID NO:60) | gcgtttgccacctgtgcaatattacttcagacggtccgcgagataacgctTTAACCCG CAACAGCAATACG |
| pntAB-cs-up (SEQ ID NO:61) | acaattatcagtctttatccggcgttctaaggtgtttatcccactatcacTGTGACGGAA GATCACTTCGCA |
| pntAB-cs-down (SEQ ID NO:62) | gcaacacgggtttcattggttaaccgttctcttggtatgccaattcgcatTTATTTGTTAA CTGTTAATTGTCCT |
| pntAB-P-up (SEQ ID NO:63) | acaattatcagtctttatccggcgttctaaggtgtttatcccactatcacTTATCTCTGG CGGTGTTGAC gcaacacgggtttcattggttaaccgttctcttggtatgccaattcgcatAGCTGTTTCC |
| pntAB-M93-down | gcaacacgggtttcattggttaaccgttctcttggtatgccaattcgcatAGCTGTTTCC |
| (SEQ ID NO:64) | TGGTTTAAACG |
| pntAB-YZ-up (SEQ ID NO:65) | tcatatcacattccttaagc |
| pntAB-YZ-down (SEQ ID NO:66) | atactttgaacttgttcttt |
| yfjb-cs-up (SEQ ID NO:67) | cattcatctcgctaacttcgcttattatggggatcagtttcagggtttcaTGTGACGGAA GATCACTTCGCA |
| yfjb-cs-down (SEQ ID NO:68) | gggtgccgtgggtgtcccacaatgccaatacacttgaaatgattattcatTTATTTGTT AACTGTTAATTGTCCT |
| yfjb-P-up (SEQ ID NO:69) | cattcatctcgctaacttcgcttattatggggatcagtttcagggtttcaTTATCTCTGGC GGTGTTGAC |
| yfjb-M37-down (SEQ ID NO:70) | gggtgccgtgggtgtcccacaatgccaatacacttgaaatgattattcatAGCTGTTT CCTGGTTTAAAC |
| yfjb-YZ-up (SEQ ID NO:71) | ttcagtacgtcgacgcaggt |
| yfjb-YZ-down (SEQ ID NO:72) | gtaatcgcatccagagaggg |
| cat-mreC-up (SEQ ID NO:73) | ggaagtgtctgtttaccctgcctggtctgatacgagaatacgcataacttTGTGACGG AAGATCACTTCGCAG |
| sacB-mreC-down (SEQ ID NO:74) | agaataaggcgaatctgtagcgacgggccacggctaaaaattggcttcatTTATTTG TTAACTGTTAATTGTCC |
| mreC-up (SEQ ID NO:75) | ggaagtgtctgtttaccctgcctggtctgatacgagaatacgcataacttatgaagccaatttt tagccg tcagtttagcgacggccaccacctgaccaacaacacctttgtcgctgatgaccAgctggc |
| mreCmut-down | tcagtttagcgacggccaccacctgaccaacaacacctttgtcgctgatgaccAgctggc |
| (SEQ ID NO:76) | cttcataaacgccattaac |
| mreC-YZ490-up (SEQ ID NO:77) | gttctctgatcggtgaagcc |
| mreC-YZ885-down (SEQ ID NO:78) | ctgcatcagacgttcattagc |

### Embodiment 1: Knockout of lactate dehydrogenase gene IdhA in MG1655 strain

It was started from wild-type *Escherichia coli* strain MG1655, a method of two-step homologous recombination was used to knock out a lactate dehydrogenase gene *IdhA,* and specific steps were as follows:

### 1.1. Construction of recombinant strain SvalM001

1) Using pXZ-CS plasmid DNA (Tan, et. al., Appl Environ Microbiol, 2013, 79:4838-4844) as a template, 2719 bp of a DNA fragment I was amplified with primers IdhA-cs-up/IdhA-cs-dwon, and used for the first step of homologous recombination.

An amplification system was: Phusion 5X buffer (NewEngland Biolabs) 10 µl, dNTP (10 mM for each dNTP) 1 µl, DNA template 20 ng, primers (10 µM) 2 µl each, Phusion High-Fidelity DNA polymerase (2.5 U/µl) 0.5 µl, distilled water 33.5 µl, and a total volume was 50 µl.

Amplification conditions were: 98°C pre-denaturation for 2 min (1 cycle); 98°C denaturation for 10 s, 56°C annealing for 10 s, 72°C extension for 2 min (30 cycles); and 72°C extension for 10 min (1 cycle).

2) The DNA fragment I was electrotransformed to the wild-type *Escherichia coli* MG1655, to obtain a SvalM001 strain.

The DNA fragment I was used for the first homologous recombination: firstly, a pKD46 plasmid (purchased from the Coil Genetic Stock Center (CGSC) of Yale University, CGSC#7739) was transformed into the wild-type *Escherichia coli* MG1655 (marked as pKD46-MG1655) by an electrotransformation method, and then the DNA fragment I was electrotransformed to the *Escherichia coli* MG1655 (pKD46-MG1655) with pKD46, to obtain a recombinant strain SvalM001.

Electrotransformation conditions were as follows: firstly, electrotransformation competent cells (a preparation method for the competent cells refers to a document: Dower et. al., 1988, Nuclear Acids Res 16:6127-6145) of the *Escherichia coli* MG1655 (marked as pKD46-MG1655) with the pKD46 plasmid were prepared; and then 50 µl of the competent cells pKD46-MG1655 were placed on ice, and 50 ng of the DNA fragment I was added, placed on ice for 2 min, and transferred to 0.2 cm of a Bio-Rad electric shock cup. A MicroPulser (Bio-Rad Company) electroporator was used, and an electric shock parameter was 2.5 kv of a voltage. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was selected for PCR verification, used primers were XZ-IdhA-up/XZ-IdhA-down, and a correct colony amplification product was 3448 bp of a fragment. A correct single colony was picked, and named as SvalM001.

### 1.2. Construction of recombinant strain SvalM002

1) Using genomic DNA of wild-type *Escherichia coli* MG1655 as a template, amplification system and amplification conditions the same as in the step 1.1 were adopted, and 476 bp of a DNA fragment II was amplified with primers XZ-IdhA-up/IdhA-del-down, and used for the second homologous recombination.

2) The DNA fragment II was electrotransformed into the recombinant strain SvalM001, to obtain a strain SvalM002.

The DNA fragment II was used for the second homologous recombination: firstly, the pKD46 plasmid was transformed into the SvalM001 (marked as pKD46-SvalM001) by the electrotransformation method, and then the DNA fragment II was electrotransformed to pKD46-SvalM001, to obtain the strain SvalM002.

Electrotransformation conditions were as follows: firstly, electrotransformation competent cells of pKD46-SvalM001 were prepared; and then 50 µl of the competent cells pKD46-SvalM001 were placed on ice, and 50 ng of a DNA fragment II was added, placed on ice for 2 min, and transferred to 0.2 cm of a Bio-Rad electric shock cup. A MicroPulser (Bio-Rad Company) electroporator was used, and an electric shock parameter was 2.5 kv of a voltage. After electric shock, 1 ml of an LB liquid medium was quickly transferred to the electric shock cup, and transferred to a test tube after blowing and beating for 5 times. It was incubated at 30°C and 75 rpm for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers XZ-ldhA-up/XZ-ldhA-down were used to perform colony PCR verification, and a correct colony amplification product was 829 bp of a fragment. A correct single colony was picked, and named as SvalM002.

Embodiment 2: Knockout of phosphoacetyl transferase encoding gene pta and acetate kinase encoding gene *ackA*

It was started from the recombinant strain SvalM002, a method of two-step homologous recombination was used to knock out a phosphoacetyl transferase encoding gene pta and an acetate kinase encoding gene *ackA*, and specific steps were as follows:

### 2.1. Construction of recombinant strain SvalM003

1) Using pXZ-CS plasmid DNA as a template, amplification system and amplification conditions the same as in the step 1.1 of Embodiment 1 were adopted, and 2719 bp of a DNA fragment I was amplified by using primers ackA-cs-up/pta-cs-down, and used for the first step of homologous recombination.
2) The DNA fragment I was electrotransformed to the strain SvalM002, to obtain a strain SvalM003.

The DNA fragment I was used for the first homologous recombination: firstly, a pKD46 plasmid was transformed into SvalM002 (marked as pKD46-SvalM002) by an electrotransformation method, and then the DNA fragment I was electrotransformed to pKD46-SvalM002.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-ackA-up/XZ-pta-down were used to perform colony PCR verification, and a correct colony amplification product should be 3351 bp. A correct single colony was picked, and named as SvalM003.

### 2.2. Construction of recombinant strain SvalM004

1) Using genomic DNA of wild-type *Escherichia coli* MG1655 as a template, amplification system and amplification conditions the same as in the step 1.1 were adopted, and 371 bp of a DNA fragment II was amplified with primers XZ-ackA-up/ackA-del-down, and used for the second homologous recombination.
2) The DNA fragment II was electrotransformed into the recombinant strain SvalM003, to obtain a strain SvalM004.

The DNA fragment II was used for the second homologous recombination: firstly, the pKD46 plasmid was transformed into the SvalM003 (marked as pKD46-SvalM003) by the electrotransformation method, and then the DNA fragment II was electrotransformed to pKD46-SvalM003, to obtain the strain SvalM004.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers XZ-ackA-up/XZ-pta-down were used to perform colony PCR verification, and a correct colony amplification product was 732 bp of a fragment. A correct single colony was picked, and named as SvalM004.

### Embodiment 3: Regulation of acetolactate synthase gene ilvBN

An artificial regulatory element M1-93 was used to regulate expression of an acetolactate synthase gene *ilvBN* by a two-step homologous recombination method, and specific steps were as follows:

### 3.1. Construction of recombinant strain SvalM005

1) Using pXZ-CS plasmid DNA as a template, amplification system and amplification conditions the same as in the step 1.1 of Embodiment 1 were adopted, and 2719 bp of a DNA fragment I was amplified with primers ilvB pro-catup/ilvB pro-catdown, and used for the first step of the homologous recombination.
2) The DNA fragment I was electrotransformed into the strain SvalM004, to obtain a strain SvaIM005.

The DNA fragment I was used for the first homologous recombination: firstly, a pKD46 plasmid was transformed into SvalM004 (marked as pKD46-SvalM004), and then the DNA fragment I was electrotransformed into pKD46-SvalM004.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers ilvB pro-YZup/ilvB pro-YZdown were used to perform colony PCR verification, and a correct colony amplification product was 2996 bp of a fragment. A correct single colony was picked, and named as SvalM005.

3.2. Construction of recombinant strain SvalM0061) Using genomic DNA of M1-93 (Lu, et. al., Appl Microbiol Biotechnol, 2012, 93:2455-2462) as a template, and 188 bp of a DNA fragment II was amplified with primers ilvB pro-up/ilvB pro-down, and used for the second homologous recombination. 2) The DNA fragment II was electrotransformed into the strain SvalM005, to obtain a strain SvalM006.

The DNA fragment II was used for the second homologous recombination: firstly, a pKD46 plasmid was transformed into SvalM005 (marked as pKD46-SvalM005), and then the DNA fragment II was electrotransformed into pKD46-SvalM005, to obtain the strain SvalM006.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers ilvB pro-YZup/ilvB pro-YZdown were used to perform colony PCR verification, and a correct colony amplification product was 465 bp of a fragment. A correct single colony was picked, and named as SvalM006.

### Embodiment 4: Regulation of acetolactate synthase gene ilvGM

An artificial regulatory element M1-93 was used to regulate expression of an acetolactate synthase gene *ilvGM* by a two-step homologous recombination method, and specific steps were as follows:

### 4.1. Construction of recombinant strain SvalM007

1) Using pXZ-CS plasmid DNA as a template, amplification system and amplification conditions the same as in the step 1.1 of Embodiment 1 were adopted, and 2719 bp of a DNA fragment I was amplified with primers ilvG pro-catup/ilvG pro-catdown, and used for the first step of the homologous recombination.
2) The DNA fragment I was electrotransformed into the strain SvalM006, to obtain a strain SvalM007.

The DNA fragment I was used for the first homologous recombination: firstly, a pKD46 plasmid was transformed into SvalM006 (marked as pKD46-SvalM006), and then the DNA fragment I was electrotransformed into pKD46-SvalM006.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers ilvG pro-YZup/ilvG p-YZdown were used to perform colony PCR verification, and a correct colony amplification product was 2993 bp of a fragment. A correct single colony was picked, and named as SvalM007.

### 4.2. Construction of recombinant strain SvalM008

1) Using genomic DNA of M1-93 (Lu, et. al., Appl Microbiol Biotechnol, 2012, 93:2455-2462) as a template, and 188 bp of a DNA fragment II was amplified with primers ilvG pro-up/ilvG pro-down, and used for the second homologous recombination.
2) The DNA fragment II was electrotransformed into the strain SvalM007, to obtain a strain SvalM008.

The DNA fragment II was used for the second homologous recombination: firstly, a pKD46 plasmid was transformed into SvalM007 (marked as pKD46-SvalM007), and then the DNA fragment II was electrotransformed into pKD46-SvalM007, to obtain the strain SvalM008.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers ilvG pro-YZup/ilvG p-YZdown were used to perform colony PCR verification, and a correct colony amplification product was 462 bp of a fragment. A correct single colony was picked, and named as SvalM008.

### Embodiment 5: Mutation of acetolactate synthase gene ilvH

A mutation was transferred into an *ilvH* gene so as to release feedback inhibition of an L-valine by a two-step homologous recombination method, and specific steps were as follows:

### 5.1. Construction of recombinant strain SvalM009

1) Using pXZ-CS plasmid DNA as a template, amplification system and amplification conditions the same as in the step 1.1 of Embodiment 1 were adopted, and 2719 bp of a DNA fragment I was amplified with primers ilvH*-cat-up/ilvH*-cat-down, and used for the first step of the homologous recombination.
2) The DNA fragment I was electrotransformed into the strain SvalM008, to obtain a strain SvalM009.

The DNA fragment I was used for the first homologous recombination: firstly, a pKD46 plasmid was transformed into SvalM008 (marked as pKD46-SvalM008), and then the DNA fragment I was electrotransformed into pKD46-SvalM008.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers ilvH*-mutYZ-up/ilvH*-mut-down were used to perform colony PCR verification, and a correct colony amplification product was 3165 bp of a fragment. A correct single colony was picked, and named as SvalM009.

### 5.2. Construction of recombinant strain SvalM010

1) Using genomic DNA of wild-type *Escherichia coli* MG1655 as a template, and 467 bp of a DNA fragment II was amplified with primers ilvH*-mut-up/ilvH*-mut-down, and used for the second homologous recombination.
2) The DNA fragment II was electrotransformed into the strain SvalM009, to obtain a strain SvalM010.

The DNA fragment II was used for the second homologous recombination: firstly, a pKD46 plasmid was transformed into SvalM009 (marked as pKD46-SvalM009), and then the DNA fragment II was electrotransformed into pKD46-SvalM009, to obtain the strain SvalM010.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers ilvH*-mutYZ-up/ilvH*-mut-down were used to perform colony PCR verification, and a correct colony amplification product was 619 bp of a fragment. A correct single colony was picked, and named as SvalM010.

Embodiment 6: Integration of NADH-dependent acetohydroxy acid isoreductase encoding gene *kari* in alcohol dehydrogenase gene *adhE* site

A acetohydroxy acid isoreductase encoding gene *kari* was obtained by whole gene synthesis after codon optimization of the kari sequence from the *Thermacetogenium phaeum* strain, as reported in the literature (Brinkmann-Chen, S., Cahn, J. K. B. & Arnold, F. H. Uncovering rare NADH-preferring ketol-acid reductoisomerases. Metab Eng 26, 17-22, doi:10.1016/j.ymben.2014.08.003 (2014).). During synthesis, an artificial regulatory element RBS5 was added in front of the *kari* gene to initiate expression of the *kari* gene, and inserted into a pUC57 vector to construct a plasmid pUC57-RBS5-kari (gene synthesis and vector construction were completed by Nanjing Genscript Biotechnology Co., Ltd.). The artificial regulatory element RBS5 and the gene *kari* were integrated into the alcohol dehydrogenase gene *adhE* site in the SvalM010 strain through a two-step homologous recombination method. Specific steps were as follows:

### 6.1. Construction of recombinant strain SvalM011

1) Using pXZ-CS plasmid DNA as a template, amplification system and amplification conditions the same as in the step 1.1 of Embodiment 1 were adopted, and 2719 bp of a DNA fragment I was amplified with primers adhE-cs-up/adhE-cs-down, and used for the first step of the homologous recombination.
2) The DNA fragment I was electrotransformed into the strain SvalM010, to obtain a strain SvalM011.

The DNA fragment I was used for the first homologous recombination: firstly, a pKD46 plasmid was transformed into SvalM010 (marked as pKD46-SvalM010), and then the DNA fragment I was electrotransformed into pKD46-SvalM010.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-adhE-up/XZ-adhE-down were used to perform colony PCR verification, and a correct colony amplification product was 3167 bp of a fragment. A correct single colony was picked, and named as SvalM011.

### 6.2. Construction of recombinant strain SvalM012

1) Using plasmid DNA pUC57-RBS5-kari as a template, and 1188 bp of a DNA fragment II was amplified with primers adhE-RBS5-up/adhE-kari-down, and used for the second homologous recombination.
2) The DNA fragment II was electrotransformed into the strain SvalM011, to obtain a strain SvalM012.

The DNA fragment II was used for the second homologous recombination: firstly, a pKD46 plasmid was transformed into SvalM011 (marked as pKD46-SvalM011), and then the DNA fragment II was electrotransformed into pKD46-SvalM011, to obtain the strain SvalM012.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers XZ-adhE-up/XZ-adhE-down were used to perform colony PCR verification, and a correct colony amplification product was 1636 bp of a fragment. A correct single colony was picked, and named as SvalM012.

### Embodiment 7: Integration of dihydroxy acid dehydratase encoding gene ilvD

It was started from SvalM012, a dihydroxy acid dehydratase encoding gene *ilvD* from *Escherichia coli* and a promoter RBSL4 controlling expression of *ilvD* were integrated into the pyruvate formate lyase encoding gene *pflB* site and substitute the *pflB* gene together by a two-step homologous recombination method, namely the *pflB* gene was knocked out while *ilvD* was integrated. Specific steps were as follows:

### 7.1. Construction of recombinant strain SvalM013

1) Using pXZ-CS plasmid DNA as a template, amplification system and amplification conditions the same as in the step 1.1 of Embodiment 1 were adopted, and 2719 bp of a DNA fragment I was amplified withprimers pfIB-CS-up/pfIB-CS-down, and used for the first step of the homologous recombination.
2) The DNA fragment I was electrotransformed into the strain SvalM012, to obtain a strain SvalM013.

The DNA fragment I was used for the first homologous recombination: firstly, a pKD46 plasmid was transformed into SvalM012 (marked as pKD46-SvalM012), and then the DNA fragment I was electrotransformed into pKD46-SvalM012.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-pflB-up600/XZ-pflB-down were used to perform colony PCR verification, and a correct colony amplification product was 3675 bp of a fragment. A correct single colony was picked, and named as SvalM013.

### 7.2. Construction of recombinant strain SvalM014

1) Using genomic DNA of wild-type *Escherichia coli* MG1655 as a template, and 1922 bp of a fragment 1 was amplified with primers ilvD-RBSL4-up/ilvD-pflB-down. A genomic DNA of M1-93 (Lu, et. al., Appl Microbiol Biotechnol, 2012, 93:2455-2462) was used as a template, and 159 bp of a DNA fragment 2 was amplified with primers RBSL4-pflB-up/ RBSL4-ilvD-down. The same PCR method as described in Embodiment 1 was adopted, 20 ng of the fragment 1 and the fragment 2 were respectively added, and primers RBSL4-pflB-up/ilvD-pflB-down were used for fusion PCR, to obtain 2040 bp of a DNA fragment II for second homologous recombination.
2) The DNA fragment II was electrotransformed into the strain SvalM013, to obtain a strain SvalM014.

The DNA fragment II was used for the second homologous recombination: firstly, a pKD46 plasmid was transformed into SvalM013 (marked as pKD46-SvalM013), and then the DNA fragment II was electrotransformed into pKD46-SvalM013, to obtain the strain SvalM014.

Electrotransformation conditions and steps were the same as the electrotransformation conditions in 2) in the step 1.2 of Embodiment 1. After electric shock, 1 ml of an LB liquid medium was quickly transferred into the electric shock cup, and transferred to a test tube after blowing and beating for 5 times, and it was incubated at 75 rpm and 30°C for 4 h. Then, bacterial solution was transferred to an LB liquid medium containing 10% sucrose without a sodium chloride (50 ml of a medium was loaded in 250 ml of a flask), and after being cultured for 24 h, it was streak-cultured on an LB solid medium containing 6% sucrose without a sodium chloride. Primers XZ-pflB-up600/XZ-pflB-down were used to perform colony PCR verification, and a correct colony amplification product was 2996 bp of a fragment. A correct single colony was picked, and named as SvalM014.

### Embodiment 8: Cloning and integration of leucine dehydrogenase encoding gene leuDH

The leucine dehydrogenase gene *leuDH* was obtained by whole gene synthesis after codon optimization of the *leuDH* sequence from *Lysinibacillus sphaericus* IFO 3525 strain, as reported in the literature (Ohshima, T. et. al., Properties of crystalline leucine dehydrogenase from Bacillus sphaericus. The Journal of biological chemistry 253, 5719-5725 (1978) ). During the synthesis, an M1-93 artificial regulatory element was added in front of the *leuDH* gene to initiate expression of the *leuDH* gene, and inserted into a pUC57 vector to construct a plasmid pUC57-M1-93-leuDH (gene synthesis and vector construction were completed by Nanjing Genscript Biotechnology Co., Ltd.). The M1-93 artificial regulatory element and the *leuDH* gene were integrated into a fumarate reductase encoding gene frd site in the SvalM014 strain through a two-step homologous recombination method and substitute the frd gene, namely the frd gene was knocked out while the *leuDH* was integrated. Specific steps were as follows:

### 8.1. Construction of recombinant strain SvalM015

1) Using pXZ-CS plasmid DNA as a template, amplification system and amplification conditions the same as in the step 1.1 of Embodiment 1 were adopted, and 2719 bp of a DNA fragment I was amplified with primers frd-cs-up/frd-cs-down, and used for the first step of the homologous recombination.
2) The DNA fragment I was electrotransformed into the strain SvalM014, to obtain a strain SvalM015.

The DNA fragment I was used for the first homologous recombination: firstly, a pKD46 plasmid was transformed into SvalM014 (marked as pKD46-SvalM014), and then the DNA fragment I was electrotransformed into pKD46-SvalM014.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-frd-up/XZ-frd-down were used to perform colony PCR verification, and a correct colony amplification product was 3493 bp of a fragment. A correct single colony was picked, and named as SvalM015.

### 8.2. Construction of recombinant strain SvalM016

1) Using plasmid DNA pUC57-M1-93-leuDH as a template, and 1283 bp of a DNA fragment II was amplified with primers frd-M93-up/frd-leuDH-down, and used for the second homologous recombination.
2) The DNA fragment II was electrotransformed into the strain SvalM015, to obtain a strain SvalM016.

The DNA fragment II was used for the second homologous recombination: firstly, a pKD46 plasmid was transformed into SvalM013 (marked as pKD46-SvalM013), and then the DNA fragment II was electrotransformed into pKD46-SvalM013, to obtain the strain SvalM014.

Electrotransformation conditions and steps were the same as the electrotransformation conditions in 2) in the step 1.2 of Embodiment 1. Primers XZ-pflB-up600/XZ-pflB-down were used to perform colony PCR verification, and a correct colony amplification product was 2057 bp of a fragment. A correct single colony was picked, and named as SvalM016.

Embodiment 9: Knockout of methylglyoxal synthase encoding gene *mgsA* and integration of NADPH-dependent acetohydroxy acid isomeroreductase encoding gene *ilvC* at themgsA site

It was started from SvalM016, the knockout of a methylglyoxal synthase encoding gene *mgsA* and the integration of a NADPH-dependent acetohydroxy acid isomeroreductase encoding gene *ilvC* at the *mgsA* site were performed through a two-step homologous recombination method. Specific steps were as follows:

### 9.1. Construction of recombinant strain SvalM017

1) Using pXZ-CS plasmid DNA as a template, amplification system and amplification conditions the same as in the step 1.1 of Embodiment 1 were adopted, and 2719 bp of a DNA fragment I was amplified with primers mgsA-cs-up/mgsA-cs-down, and used for the first step of the homologous recombination.
2) The DNA fragment I was electrotransformed into the strain SvalM016, to obtain a strain SvalM017.

The DNA fragment I was used for the first homologous recombination: firstly, a pKD46 plasmid was transformed into SvalM016 (marked as pKD46-SvalM016), and then the DNA fragment I was electrotransformed into *Escherichia coli* SvalM016 with pKD46 (pKD46-SvalM016).

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-mgsA-up/XZ-mgsA-down were used to perform colony PCR verification, and a correct colony amplification product was 3646 bp of a fragment. A correct single colony was picked, and named as SvalM017.

### 9.2. Construction of recombinant strain SvalM018

1) Using DNA of wild-type *Escherichia coli* MG1655 as a template, and 566 bp of a DNA fragment II was amplified with primers XZ-mgsA-up/mgsA-del-down, and used for the second homologous recombination.
2) The DNA fragment II was electrotransformed into the strain SvalM017 with pKD46 (marked as pKD46-SvalM017), to obtain a strain SvalM018. Electrotransformation conditions and steps were the same as the electrotransformation conditions in 2) in the step 1.2 of Embodiment 1. Primers XZ-mgsA-up/XZ-mgsA-down were used to perform colony PCR verification, and a correct colony amplification product was 1027 bp of a fragment. A correct single colony was picked, and named as SvalM018.

### 9.3. Construction of recombinant strain SvalM019

1) Using DNA of wild-type *Escherichia coli* MG1655 as a template, and 1547 bp of a fragment 1 was amplified with primers ilvC-M46-up/ ilvC-mgsA-down. A genomic DNA of M1-46 (Lu, et. al., Appl Microbiol Biotechnol, 2012, 93:2455-2462) was used as a template, and 160 bp of a fragment 2 was amplified with primers M46-mgsA-up/ M46-ilvC-down. The same PCR method as described in Embodiment 1 was adopted, 20 ng of the fragment 1 and the fragment 2 were respectively added, and primers M46-mgsA-up/ilvC-mgsA-down were used for fusion PCR, to obtain 1664 bp of a DNA fragment II for second homologous recombination.
2) The DNA fragment II was electrotransformed into the strain SvalM017 with pKD146, to obtain a strain SvalM019. Electrotransformation conditions and steps were the same as the electrotransformation conditions in 2) in the step 1.2 of Embodiment 1. Primers XZ-mgsA-up/XZ-mgsA-down were used to perform colony PCR verification, and a correct colony amplification product was 2591 bp of a fragment. A correct single colony was picked, and named as SvalM019.

### Embodiment 10: Regulation of transhydrogenase gene pntAB

It was started from SvalM019, an artificial regulatory element was used to regulate expression of a transhydrogenase gene *pntAB*, thereby activate the activity of the PntAB. Specific steps were as follows:

### 10.1. Construction of recombinant strain SvalM020

1) Using pXZ-CS plasmid DNA as a template, amplification system and amplification conditions the same as in the step 1.1 of Embodiment 1 were adopted, and 2719 bp of a DNA fragment I was amplified with primers pntAB-cs-up/pntAB-cs-down, and used for the first step of the homologous recombination.
2) A pKD46 plasmid was transformed into SvalM019 (marked as pKD46-SvalM019) by an electrotransformation method, and the DNA fragment I was electrotransformed into the strain pKD46-SvalM019, to obtain a strain SvalM020.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers pntAB-YZ-up/pntAB-YZ-down were used to perform colony PCR verification, and a correct colony amplification product was 3459 bp of a fragment. A correct single colony was picked, and named as SvaIM020.

### 10.2. Construction of recombinant strain SvalM021

1) Using genomic DNA of M1-93 as a template, and 188 bp of a DNA fragment II was amplified with primers pntAB-P-up/pntAB-M93-down, and used for the second homologous recombination.
2) A pKD46 plasmid was transformed into SvalM020 (marked as pKD46-SvalM020) by an electrotransformation method, and the DNA fragment II was electrotransformed into the strain pKD46-SvaIM020, to obtain a strain SvaIM021.

Electrotransformation conditions and steps were the same as the electrotransformation conditions in 2) in the step 1.2 of Embodiment 1. Primers pntAB-YZ-up/pntAB-YZ-down were used to perform colony PCR verification, and a correct colony amplification product was 928 bp of a fragment. A correct single colony was picked, and named as SvaIM021.

### Embodiment 11: Regulation of NAD kinase encoding gene yfjB

It was started from SvaIM021, an artificial regulatory element was used to regulate expression of a NAD kinase gene *yfjB,* thereby activate the activity of the YfjB, so that achieving cofactor supply balance by working together transhydrogenase and NAD kinase. Specific steps were as follows:

### 11.1. Construction of recombinant strain SvalM022

1) Using pXZ-CS plasmid DNA as a template, amplification system and amplification conditions the same as in the step 1.1 of Embodiment 1 were adopted, and 2719 bp of a DNA fragment I was amplified with primers yfjb-cs-up/yfjb-cs-down, and used for the first step of the homologous recombination.
2) A pKD46 plasmid was transformed into SvalM021 (marked as pKD46-SvalM021) by an electrotransformation method, and the DNA fragment I was electrotransformed into the strain pKD46-SvaIM021, to obtain a strain SvaIM022.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB solid plate containing ampicillin (a final concentration was 100 µg/ml) and chloramphenicol (a final concentration was 34 µg/ml). After being cultured overnight at 30°C, a single colony was picked, primers yfjb-YZ-up/yfjb-YZ-down were used to perform colony PCR verification, and a correct colony amplification product was 3542 bp of a fragment. A correct single colony was picked, and named as SvaIM022.

### 11.2. Construction of recombinant strain SvalM023

1) Using genomic DNA of M1-37 (Lu, et. al., Appl Microbiol Biotechnol, 2012, 93:2455-2462) as a template, and 188 bp of a DNA fragment II was amplified with primers yfjb-P-up/ yfjb-M37-down, and used for the second homologous recombination.
2) A pKD46 plasmid was transformed into SvalM022 (marked as pKD46-SvaIM022) by an electrotransformation method, and the DNA fragment II was electrotransformed into the strain pKD46-SvaIM022, to obtain a strain SvalM023.

Electrotransformation conditions and steps were the same as the electrotransformation conditions in 2) in the step 1.2 of Embodiment 1. Primers yfjb-YZ-up/yfjb-YZ-down were used to perform colony PCR verification, and a correct colony amplification product was 1011 bp of a fragment. A correct single colony was picked, and named as SvalM023.

### Embodiment 12: Knockout of alcohol dehydrogenase gene adhE site

It was started from SvalM023, and a two-step homologous recombination method was used to knock out an alcohol dehydrogenase gene *adhE.* Specific steps were as follows:

### 12.1. Construction of recombinant strain SvalM024

1) Using pXZ-CS plasmid DNA as a template, amplification system and amplification conditions the same as in the step 1.1 of Embodiment 1 were adopted, and 2719 bp of a DNA fragment I was amplified with primers adhE-cs-up/adhE-cs-down, and used for the first step of the homologous recombination.
2) A pKD46 plasmid was transformed into SvalM023 (marked as pKD46-SvalM023) by an electrotransformation method, and the DNA fragment I was electrotransformed into the strain pKD46-SvalM023, to obtain a strain SvalM024.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers XZ-adhE-up/XZ-adhE-down were used to perform colony PCR verification, and a correct colony amplification product was 3167 bp of a fragment. A correct single colony was picked, and named as SvalM024.

### 12.2. Construction of recombinant strain SvalM025

1) Using DNA of wild-type *Escherichia coli* MG1655 as a template, and 271 bp of a DNA fragment II was amplified with primers XZ-adhE-up/adhE-del-down, and used for the second homologous recombination.
2) A pKD46 plasmid was transformed into SvalM024 (marked as pKD46-SvalM024) by an electrotransformation method, and the DNA fragment II was electrotransformed into the strain pKD46-SvaIM024, to obtain a strain SvalM025.

Electrotransformation conditions and steps were the same as the electrotransformation conditions in 2) in the step 1.2 of Embodiment 1. Primers XZ-adhE-up/XZ-adhE-down were used to perform colony PCR verification, and a correct colony amplification product was 548 bp of a fragment. A correct single colony was picked, and named as SvalM025.

### Embodiment 13: Introduction of genomic mreC point mutation

It was started from the strain SvalM018, a point mutation was transferred at the 449-th site in an encoding region of a wild-type mreC gene shown in SEQ IDNO: 1 by a two-step homologous recombination method, and C was mutated into T (the nucleotide sequence after mutation was shown in SEQ ID NO: 4), correspondingly the 150-th amino acid, encoded by it, of an EcoIC_0457 rod-shaped determining protein shown in SEQ ID NO: 2 was mutated from Proline (P) to Leucine (L) (the amino acid sequence after mutation was shown in SEQ ID NO: 3), to obtain a strain SvaIM027. Specific steps were as follows:

### 13.1. Construction of recombinant strain SvalM026

1) Using pXZ-CS plasmid DNA as a template, amplification system and amplification conditions the same as in the step 1.1 of Embodiment 1 were adopted, and 2719 bp of a DNA fragment I was amplified with primers cat-mreC-up/SacB-mreC-down, and used for the first step of the homologous recombination.
2) A pKD46 plasmid was transformed into SvalM018 (marked as pKD46-SvalM018) by an electrotransformation method, and the DNA fragment I was electrotransformed into the strain pKD46-SvalM018, to obtain a strain SvalM026.

Electrotransformation conditions and steps were the same as the electrotransformation conditions in 2) in the step 1.1 of Embodiment 1. After electric shock, 1 ml of an LB liquid medium was quickly transferred into the electric shock cup, and transferred to a test tube after blowing and beating for 5 times, and it was incubated at 75 rpm and 30°C for 2 h. 200 µl of bacterial solution was taken and applied on an LB plate containing ampicillin (a final concentration was 100 µg/ml) and chloramphenicol (a final concentration was 34 µg/ml). After being cultured overnight at 30°C, a single colony was picked, primers mreC-YZ490-up/mreC-YZ885-down were used to perform colony PCR verification, and a correct colony amplification product was 3994 bp of a fragment. A correct single colony was picked, and named as SvalM026.

### 13.2. Construction of recombinant strain SvalM027

1) Using DNA of wild-type *Escherichia coli* MG1655 as a template, and 552 bp of a DNA fragment II was amplified with primers mreC-up/mreCmut-down, and used for the second homologous recombination.
2) A pKD46 plasmid was transformed into SvalM026 (marked as pKD46-SvalM026) by an electrotransformation method, and the DNA fragment II was electrotransformed into the strain pKD46-SvalM026, to obtain a strain SvaIM027.

Herein, electrotransformation conditions and steps were the same as the electrotransformation conditions in 2) in the step 1.2 of Embodiment 1. Primers mreC-YZ490-up/mreC-YZ885-down were used to perform colony PCR verification, and a correct colony amplification product was 1375 bp of a fragment. A correct single colony was picked, and named as SvaIM027.

It was started from the strain SvaIM025, a point mutation was transferred at the 449-th site in an encoding region of a wild-type mreC gene shown in SEQ IDNO: 1 by using the same method as SvaIM027, and C was mutated into T (the nucleotide sequence after mutation was shown in SEQ ID NO: 4), correspondingly the 150-th amino acid, encoded by it, of an EcoIC_0457 rod-shaped determining protein shown in SEQ ID NO: 2 was mutated from Proline (P) to Leucine (L) (the amino acid sequence after mutation was shown in SEQ ID NO: 3), to obtain a recombinant strain SvaIM029. Specific steps were as follows:

### 13.3. Construction of recombinant strain SvalM028

1) Using pXZ-CS plasmid DNA as a template, amplification system and amplification conditions the same as in the step 1.1 of Embodiment 1 were adopted, and 2719 bp of a DNA fragment I was amplified with primers cat-mreC-up/SacB-mreC-down, and used for the first step of the homologous recombination.
2) A pKD46 plasmid was transformed into SvalM025 (marked as pKD46-SvalM025) by an electrotransformation method, and the DNA fragment I was electrotransformed into the strain pKD46-SvaIM025, to obtain a strain SvalM028.

Herein, electrotransformation conditions and steps were the same as the electrotransformation conditions in 2) in the step 1.1 of Embodiment 1. Primers mreC-YZ490-up/mreC-YZ885-down were used to perform colony PCR verification, and a correct colony amplification product was 3994 bp of a fragment. A correct single colony was picked, and named as SvalM028.

### 13.4. Construction of recombinant strain SvalM029

1) Using DNA of wild-type *Escherichia coli* MG1655 as a template, and 552 bp of a DNA fragment II was amplified with primers mreC-up/mreCmut-down, and used for the second homologous recombination.
2) A pKD46 plasmid was transformed into SvalM028 (marked as pKD46-SvalM028) by an electrotransformation method, and the DNA fragment II was electrotransformed into the strain pKD46-SvalM028, to obtain a strain SvaIM029.

Herein, electrotransformation conditions and steps were the same as the electrotransformation conditions in 2) in the step 1.2 of Embodiment 1. Primers mreC-YZ490-up/mreC-YZ885-down were used to perform colony PCR verification, and a correct colony amplification product was 1375 bp of a fragment. A correct single colony was picked, and named as SvaIM029.

### Embodiment 14: Production of L-valine by fermentation of recombinant strain

A seed culture medium was formed by the following components (a solvent was water):
Macroelement: 20 g/L of glucose, 0.87 g/L of NH₄H₂PO₄, 2.63 g/L of (NH₄) ₂HPO₄, 0.18 g/L of MgSO₄·7H₂O, and 0.15 g/L of betaine-HCl.
Microelement: 1.5 µg/L of FeCl₃·6H₂O, 0.1 µg/L of CoCl₂·6H₂O, 0.1 µg/L of CuCl₂·2H₂O, 0.1 µg/L of ZnCl₂, 0.1 µg/L of Na₂MoO₄·2H₂O, 0.2 µg/L of MnCl₂·4H₂O, and 0.05 µg/L of H₃BO₃.

The fermentation culture medium was mostly the same as the seed culture medium, and a difference was only that the glucose concentration was 50 g/L.

The anaerobic fermentation of the strain SvalM029 to produce the L-valine involves the following steps:
(1) Seed culture: a fresh clone on an LB solid plate was inoculated into a test tube containing 4 ml of a seed culture medium, cultivated overnight at 37°C with shaking at 250 rpm. Then, a culture was transferred to a 250 ml triangular flask containing 30 ml of the seed culture medium according to an inoculum size of 2% (V/V), and cultivated for 12 h at 37°C with shaking at 250 rpm to obtain seed culture solution for inoculation of the fermentation culture medium.
(2) Fermentation culture: in a 500 ml anaerobic tank, the volume of the fermentation culture medium was 250 ml, the seed culture solution was inoculated into the fermentation culture medium according to an inoculum size of final concentration OD550=0.1, and fermented for 72 h at 37°C with shaking at 150 rpm, to obtain fermentation solution.

Herein, a neutralizer used was 7 M ammonia water, so pH of the fermentation tank was controlled at 7.0, and any gases were not allowed in a culture process.

Analysis method: an Agilent-1260 high-performance liquid chromatograph was used to determine the components of the fermentation solution after 72 h of fermentation. The concentrations of glucose and organic acids in the fermentation solution were determined by using an Aminex HPX-87H organic acid analysis column from Biorad Company. The amino acid was determined by using a Sielc amino acid analysis column primesep 100 250 × 4.6 mm.

The same fermentation method mentioned above was adopted, the strain SvalM018, the strain SvalM027 and the strain SvalM025 were respectively used for anaerobic fermentation production and analysis detection of the L-valine, and results were shown in Table 3.

**Table 3: Production of valine by anaerobic fermentation of recombinant strain**

| Strain | OD550 | Valine yield (g/L) |
|---|---|---|
| SvalM018 | 1.03 | 1.15 |
| SvalM027 | 2.1 | 4.5 |
| SvalM025 | 1.01 | 1.2 |
| SvalM029 | 2.8 | 5.1 |

It may be seen from Table 3 that for the valine engineering bacteria (recombinant strain SvalM018) that combines NADH-dependent KARI and NADH-dependent LeuDH, the yield of the valine may be increased by 291% after the genome point mutation (recombinant strain SvalM027) was transferred; and for the valine engineering bacteria (recombinant strain SvalM025) that combines NADPH-dependent IIvC, NADH-dependent LeuDH, transhydrogenase PntAB and NAD kinase YfjB, the yield of the valine may be increased by 325% after the genome point mutation (recombinant strain SvalM029) was transferred. In conclusion, the introduction of the point mutation of mreC into the genome may effectively improve the production capacity of the valine engineering bacteria, while the cell biomass was also significantly improved.

The above are only preferred embodiments of the present disclosure, and a scope of protection of the present disclosure is not limited to the above embodiments. All technical schemes under the ideas of the present disclosure belong to the scope of protection of the present disclosure. It should be pointed out that for those of ordinary skill in the field, a plurality of improvements and modifications made without departing from the principles of the present disclosure should be considered as the scope of protection of the present disclosure.

## Claims

1. An mreC mutant, wherein it is a protein composed of an amino acid sequence shown in SEQ ID NO: 3.

2. The mreC mutant according to claim 1, wherein the mreC mutant is a protein obtained after the proline at the 150-th site of a rod-shaped determining protein amino acid sequence shown in SEQ ID NO: 2 is mutated into leucine.

3. A nucleic acid molecule encoding the mreC mutant according to claim 1.

4. The nucleic acid molecule according to claim 3, wherein the nucleic acid molecule is a deoxyribonucleic acid (DNA) molecule described in (a1) or (a2):
(a1) a DNA molecule of which an encoding region is shown in SEQ ID NO: 4; and
(a2) a DNA molecule of which a nucleotide sequence is shown in SEQ ID NO: 4,
wherein the DNA molecule shown in SEQ ID NO: 4 is that C at the 449-th site of a nucleotide sequence in an encoding region of a wild-type mreC gene shown in SEQ ID NO: 1 is mutated into T.

5. A biomaterial, wherein it is any one of the following (b1)-(b3):
(b1) a gene expression cassette containing the nucleic acid molecule according to claim 3 or 4;
(b2) a recombinant vector containing the nucleic acid molecule according to claim 3 or 4; and
(b3) a recombinant microorganism containing the nucleic acid molecule according to claim 3 or 4.

6. Use of any one of the following (c1)-(c2):
(c1) use of the mreC mutant according to claim 1 or 2, the nucleic acid molecule according to claim 3 or 4, or the biomaterial according to claim 5 in construction of L-valine engineering bacteria; and
(c2) use of the mreC mutant according to claim 1 or 2, the nucleic acid molecule according to claim 3 or 4, or the biomaterial according to claim 5 in production of L-valine.

7. A method for constructing L-valine engineering bacteria, wherein it comprises the following steps: transferring the nucleic acid molecule according to claim 3 or 4 into a microorganism.

8. The method according to claim 7, wherein it further comprises performing the following modifications (d1) or (d2):
(d1) transferring a NADH-dependent acetohydroxy acid isoreductase gene *kari* and a NADH-dependent leucine dehydrogenase gene *leuDH* into the recombinant microorganism according to claim 7, thereby enhancing the enzyme activity; and
(d2) transferring a NADPH-dependent acetohydroxy acid isomeroreductase gene *ilvC* and a NADH-dependent leucine dehydrogenase gene *leuDH* into the recombinant microorganism according to claim 7, and activating the activity of a transhydrogenase PntAB and the activity of a NAD kinase YfjB, thereby enhancing the enzyme activity.

9. The method according to claim 8, wherein it further comprises the following modifications (1)-(6) to the recombinant microorganism:
(1) knocking out a lactate dehydrogenase gene *IdhA*;
(2) knocking out a phosphoacetyl transferase gene pta and an acetate kinase gene *ackA*;
(3) knocking out an alcohol dehydrogenase gene *adhE*;
(4) knocking out a fumarate reductase gene frd and a pyruvate formate lyase gene *pflB*;
(5) knocking out a methylglyoxal synthase gene *mgsA*; and
(6) enhancing the activities of an acetolactate synthase (AHAS) and a dihydroxy acid dehydratase (*ilvD*).

10. The method according to claim 9, wherein the microorganism is *Escherichia coli;* and preferably, the microorganism is *Escherichia coli* MG1655.

11. The method according to claim 9, wherein AHAS comprises IIvBN, IIvGM, and IIvIH;
preferably, the activity of IIvIH is enhanced by relieving the feedback inhibition of valine on IIvH; and
more preferably, the activity of IIvIH is enhanced by mutating a *ilvH* gene.

12. The L-valine engineering bacteria obtained by the method according to any one of claims 7-11.

13. Use of the L-valine engineering bacteria according to claim 12 in L-valine production.

14. A method for producing an L-valine, comprising the following steps:
(1) culturing the L-valine engineering bacteria according to claim 12; and
(2) separating and harvesting the L-valine.
